# EUROPEAN PATENT APPLICATION

(11) **EP 4 721 735 A1**
(43) Date of publication of application: **08.04.2026**
(21) Application number: 24204273.7
(22) Date of filing: 02.10.2024
(51) Int. Cl.: A61K 31/19, A61K 31/195, A61K 38/28, A61K 38/50, A61K 38/54, A61P 35/00, A23L 29/00, A23L 33/00, A23L 33/175

(54) **COMPOSITION FOR ENTERAL NUTRITION OF CANCER PATIENTS**

(71) Applicant: Kyon Biotech AG, 8005 Zürich (CH)
(72) Inventor: TEPIC, Slobodan, 8057 Zürich (CH); CVETKOVIC, Goran, 8645 Jona (CH); CVETKOVIC, Olivera, 8645 Jona (CH)
(74) Representative: Weickmann & Weickmann PartmbB

(57) **Abstract**

The present invention relates to a treatment of cancer combining metabolic with enzymatic means of selective attack on cancer cells.

## Description

The present invention relates to compositions for enteral nutrition of cancer patients undergoing amino acid depletion as a therapeutical modality.

### Background

Depletion of L-arginine (in the following arginine) has been shown of utility in treating some cancers such as hepatocellular carcinoma and melanoma, and based on *in vitro* work, probably many others. The use of arginine-depleting enzymes such as arginase in cancer therapy has e.g., been described by Shen et al. (Cell Death & Disease 8 (2017), e2720), Zou et al. (Biomedicine & Pharmacotherapy 118 (2019), 109210), Al-Koussa et al. (Cancer Cell International 20 (2020) Article number 150) and Zhang et al. (Cancer Letters 502 (2012), 58-70), the contents of which are herein incorporated by reference. In a recent publication with inventors as co-authors, Chew HY et al. (Arginase-induced cell death pathways and metabolic changes in cancer cells are not altered by insulin. Sci Rep. 2024 Feb 19;14(1):4112. doi: 10.1038/s41598-024-54520-z. PMID: 38374190; PMCID: PMC10876525.), the utility of arginine depletion was shown against 9 cancer lines, 3 of each of the breast, lung, and ovary. Four of these nine cancer lines could be completely eliminated when treated with recombinant human liver arginase at 1 IU/ml; the other five with 10 IU/ml during 9 days. This *in vitro* study showed that achieving the level of enzymatic activity and duration is of fundamental importance for the successful treatment of cancer.

The use of arginine-depleting enzymes is necessary, but our own research has shown it is not sufficient to cause and maintain systemic, deep depletion of arginine needed to cause rapid, selective killing of cancer cells.

The use of an insulin/glucose clamp in parallel with the enzymatic degradation of arginine makes the task of deep arginine depletion much more manageable.

Insulin is a growth factor and thus promotes protein synthesis and inhibits protein breakdown. This is of crucial importance when the task is removing an amino acid from circulation, particularly removing arginine, which is a semi-essential amino acid under tight homeostatic control.

An increase of vascular permeability by insulin also helps in getting therapeutic enzymes into interstitial fluid space, closer to where most cancerous cells reside. Finally, insulin may also play a role in transporting arginine-degrading enzymes into cancerous cells by stimulating endocytosis.

However, as the inventors' research has shown, even with the use of an insulin/glucose clamp, free arginine levels could only be reduced to about 5 to 10 µM, i.e., to about 5 to 10% of the normal plasma concentration of about 100 µM. At these free arginine levels, cells, cancerous and healthy, will not proliferate but still can survive for prolonged periods. The target for arginine concentration to result in rapid killing of cancer cells is 1 µM or less. Healthy cells can survive such low levels of arginine much longer than cancer cells (weeks vs days) which accounts for the selectivity of this intervention.

The main obstacle to achieving this level of arginine depletion is the conversion of citrulline into arginine by the kidneys. Citrulline is produced by intestinal lining cells from ornithine which in turn is produced from non-essential amino acids glutamine and proline.

The inventors have attempted inhibition of citrulline production in the intestines but none of the many approaches tested in experimental dogs have resulted in the satisfactory reduction of plasma citrulline.

The resolution to the problem of citrulline as a precursor of arginine was found in the use of argininosuccinate synthase (ASS) which converts citrulline and aspartate to argininosuccinate. ASS is delivered to the vascular system where it interrupts the transport of citrulline from the intestines to the kidneys. In the urea cycle of the liver, argininosuccinate is converted to arginine by argininosuccinate lyase (ASL) but this does not happen in the vascular system. Accordingly, WO 2023/066910, the content of which is herein incorporated by reference, describes a medicament comprising an arginine decomposing enzyme such as arginase (ARG) and a citrulline converting enzyme such as argininosuccinate synthase (ASS) for the treatment of cancer.

Certain blood cancers, e.g., acute lymphoblastic leukemia (ALL) or non-Hodgkin lymphoma, are highly susceptible to depletion of asparagine, a non-essential amino acid. The use of L-asparaginase (ASNase) in treating these blood cancers is a highly successful precedent for all other amino-acid depletion schemes. Unlike arginine, asparagine is easily depleted from circulation due to the apparent lack of any systemic mechanisms of homeostasis. L-asparaginase also degrades glutamine, another non-essential amino acid. Glutamine is used as a precursor for many biosynthetic pathways and is found in abundance in all cells and all extracellular fluids.

WO 2020/245041, the content of which is herein incorporated by reference, discloses delivery of asparaginase (ASNase) in a dissociated form, particularly in the form of monomeric units.

WO 2022/034218, the content of which is herein incorporated by reference, discloses that the efficacy of biologics may be increased by assisting the extravasation of these compounds by co-administering a biological therapeutic molecule together with insulin and glucose.

EP 23206427.9, the content of which is herein incorporated by reference, discloses the treatment of cancer by amino acid depletion, wherein at least one amino acid-degrading enzyme and a blood glucose-lowering agent are administered under conditions of glucose depletion. The treatment may further comprise administering a ketone body compound, e.g., a physiologically acceptable 3-hydroxybutyrate salt.

EP 24182531.4, the content of which is herein incorporated by reference, discloses the use of a hypotonic fluid, e.g., water for injection, for the treatment of cancer by amino acid depletion via the endogenous release of liver enzymes, including liver arginase, by infusion of said fluid into the hepatic vein of a liver segment.

EP 24186856.1, the content of which is herein incorporated by reference, discloses the use of endogenous liver enzymes in the treatment of cancer by amino acid depletion, wherein the liver enzymes are released by liver reperfusion injury caused by temporary occlusion of a hepatic vein of a liver segment. The treatment may further comprise administering glucose and/or a mixture of essential amino acids without arginine.

The present inventors have found that the combined catalytic activity of the amino acid degrading enzymes ARG, ASS, and ASNase together with insulin but under conditions of glucose depletion leads to the systemic reduction of arginine, citrulline, asparagine, and glutamine. Most cancer cells are rapidly - in a matter of one or a few days - eliminated while the healthy cells survive with minimal losses. Healthy, normally proliferating cells such as those of the intestinal lining or bone marrow, exit the cycle but can resume proliferation once these amino acids are brought back to normal physiological levels.

It was an object of the present invention to provide a means for providing an improved treatment schedule in the depletion of free amino acids including arginine, citrulline and/or asparagine in the blood of a subject, particularly of a cancer patient.

More particularly, it was an object of that invention to overcome disadvantages associated with previous treatment schedules involving amino acid depletion with, e.g., administration of PEGylated arginase (ARG) or PEGylated arginine deiminase (ADI), which are currently investigated in over 30 clinical trials for cancer treatment.

### Summary of the invention

The present inventors have found that the combined catalytic activity of the three enzymes ARG, ASS, and ASNase together with insulin optionally under conditions of glucose depletion leads to the systemic reduction of arginine, citrulline, asparagine, and glutamine. Most cancer cells are rapidly - in a matter of one or a few days - eliminated while the healthy cells survive with minimal losses. Healthy, normally proliferating cells such as those of the intestinal lining or bone marrow, exit the cycle but can resume proliferation once these amino acids are brought back to normal physiological levels.

The cancer treatment of the present invention particularly comprises a systemic amino acid depletion, i.e., an amino acid depletion throughout the subject's body. The efficacy of an amino acid depletion can be improved by enterally administering a nutritional composition to a subject to be treated.

A first aspect of the present invention relates to a nutritional composition for enteral administration for use in a method of treating cancer by amino acid depletion, wherein the composition comprises (i) at least one ketone body compound, e.g., at least one beta-hydroxybutyrate (BHB) and (ii) a mixture of essential amino acids.

This aspect also relates to a method for the treatment of cancer by amino acid depletion, wherein a nutritional composition comprising (i) at least one ketone body compound, e.g., at least one beta-hydroxybutyrate (BHB) and (ii) a mixture of essential amino acids is administered enterally to a subject in need thereof.

This aspect also refers to a medicament comprising or consisting of a combination of the following active agents:
(i) a nutritional composition for enteral administration comprising at least one ketone body compound, e.g., a physiologically acceptable 3-hydroxybutyrate and a mixture of essential amino acids, particularly 9 essential amino acids optionally together with L-aspartate;
(ii) a blood glucose-lowering agent, e.g., an insulin;
(iii) an arginine-decomposing enzyme such as an ARG;
(iv) a citrulline-converting enzyme such as an ASS,
(v) an asparagine-decomposing enzyme such as an ASNase; and
(vi) optionally a glutamine-decomposing enzyme such as a GLS.

In particular embodiments, the medicament is for use in a method of treating cancer, more particularly in a method of treating cancer by amino acid depletion.

A second aspect of the present invention relates to a nutritional composition for enteral administration for use in a method of treating cancer by amino acid depletion, wherein the composition comprises a mixture of essential amino acids. In this aspect, the composition may be free from a ketone body compound, e.g., free from a BHB.

This aspect of the present invention also relates to a method for the treatment of cancer by amino acid depletion, wherein a nutritional composition comprising a mixture of essential amino acids is administered enterally to a subject in need thereof.

This aspect also refers to a medicament comprising or consisting of a combination of the following active agents:
(i) a nutritional composition for enteral administration comprising a mixture of essential amino acids, particularly 9 essential amino acids optionally together with L-aspartate;
(ii) a blood glucose-lowering agent, e.g., an insulin;
(iii) an arginine-decomposing enzyme such as an ARG;
(iv) a citrulline-converting enzyme such as an ASS,
(v) an asparagine-decomposing enzyme such as an ASNase; and
(vi) optionally a glutamine-decomposing enzyme such as a GLS.

In particular embodiments, the medicament is for use in a method of treating cancer, more particularly in a method of treating cancer by amino acid depletion.

In certain embodiments, the enteral nutritional composition may further comprise one or more of a carbohydrate source, a lipid source, and a source of fibers. Furthermore, the nutritional composition optionally comprises water.

The enteral nutritional composition may be co-administered together with further agents including insulin and optionally an NO donor and/or a pressor peptide. The further agents are typically delivered separately from the nutritional composition, e.g., subcutaneously, intravenously, transdermally or orally.

In certain embodiments, amino acid depletion comprises the administration of at least one exogenous amino acid degrading enzyme to the subject. In particular embodiments, the exogenous amino acid-degrading enzyme is selected from:
- an asparaginase, particularly in a dissociated form, more particularly in the form of monomers;
- an arginase;
- an argininosuccinate synthase;
- a glutaminase;
- or any combination thereof.

In certain embodiments, amino acid depletion comprises targeted disruption of cells within the subject's body thereby releasing at least one endogenous amino acid degrading enzyme. In particular embodiments, the endogenous amino acid-degrading enzyme, e.g., arginase and/or argininosuccinate synthase is provided endogenously by liver cells and/or erythrocytes due to their low glucose-induced necrosis. Hepatocytes and erythrocytes are the only known healthy cells that cannot utilize BHBs as an alternative energy source. This peculiar metabolic feature of hepatocytes (and erythrocytes which also contain a significant amount of arginase type II) enables high BHB-low glucose conditions to augment and potentiate the so-called metabolic treatment of cancers by amino acid depletion, particularly by arginine depletion.

In certain embodiments, the release of endogenous amino acid degrading enzymes, particularly ARG and ASS, from disrupted liver cells is effected by the administration of a hypotonicfluid, e.g., water for injection, by infusion of said fluid into the hepatic vein of a liver segment, e.g., as described in EP 24182531.4, supra.

In certain embodiments, the release of endogenous amino acid degrading enzymes, particularly ARG and ASS, from disrupted liver cells is effected by liver reperfusion injury caused by temporary occlusion of a hepatic vein of a liver segment, e.g. as described in EP 24186856.1, supra.

In particular embodiments, the amino acids depleted are L-arginine and L-glutamine, together with L-citrulline and L-asparagine. The preferred enzyme for the degradation of L-arginine is arginase (ARG). The preferred enzyme for the degradation of L-asparagine is asparaginase (ASNase), which converts L-asparagine to L-aspartic acid but also L-glutamine to L-glutamic acid.

Our experimental clinical studies with ASNase for canine lymphoma have shown variable effectiveness of ASNase in lowering systemic levels of glutamine. It is therefore an option to administer glutaminase as a further enzyme. Our recent in vitro research gives a strong rationale for combining these enzymes. Exposed to arginase, cancer cells have shown decreased intracellular concentrations of all amino acids but those of asparagine and glutamine have increased. While it is not clear why, it seems reasonable to assume that removing these two amino acids with asparaginase and glutaminase should have a synergetic effect with arginase.

In certain embodiments, the amino acid depletion is combined with the depletion of glucose. In these embodiments, the present invention combines two powerful attacks on cancer cells - systemic depletion of amino acids for protein synthesis and depletion of glucose.

In particular embodiments, glucose is systemically lowered by the use of a glucose-lowering agent such as insulin. The lack of glucose may be compensated by the administration of ketone body compounds, e.g., BHBs such as physiologically acceptable BHB salts including but not limited to sodium, calcium, potassium, magnesium and/or lysine beta-hydroxybutyrate. Further examples of BHBs are physiologically acceptable BHB esters including but not limited to 3-hydroxybutyl-3-hydroxybutyrate or ethyl-3-hydroxybutyrate.

### Description of preferred embodiments

According to the present invention, a subject in need thereof is subjected to a treatment with at least one amino acid-degrading enzyme and optionally a blood glucose-lowering agent under conditions of glucose depletion.

The term "amino acid-degrading enzyme" relates to an enzyme, which is capable of reducing the amount of an amino acid under physiological conditions. In certain embodiments, the present invention comprises a treatment involving reducing the amount of amino acids selected from L-arginine, L-citrulline, L-asparagine and L-glutamine in order to provide conditions that are detrimental to the survival of cancer cells within the body of a subject to be treated.

In certain embodiments, the amino acid depletion comprises the depletion of L-arginine or the depletion of L-arginine and L-citrulline.

In certain embodiments, the amino acid depletion comprises the depletion of L-asparagine or the depletion of L-asparagine and L-glutamine.

In certain embodiments, the amino acid depletion comprises the depletion of L-arginine, L-citrulline and L-asparagine.

In certain embodiments, the amino acid depletion comprises the depletion of L-arginine, L-citrulline, L-asparagine and L-glutamine.

In certain embodiments, the present invention relates to the depletion of L-arginine by enzymatic means, particularly for the treatment of cancer, in combination with the concurrent depletion of glucose and L-glutamine.

The present invention comprises enteral administration of a nutritional composition during the amino acid depletion treatment.

In certain embodiments, the nutritional composition has a nutritional value of about 300 to about 600 kcal per 100 g dry weight.

The composition may be a liquid composition comprising the active agent(s) in dissolved or suspended form ready for use. Alternatively, the composition may be a solid composition comprising the active agent(s) in lyophilized or freeze-dried form for reconstitution with a suitable solvent, e.g., an aqueous solvent. In certain embodiments, the nutritional composition is a liquid composition having a content of dry matter of about 10% to about 60% (w/w), about 20% to about 40%(w/w) or about 30% (w/w) based on the total weight of the composition.

According to the first aspect of the present invention, the composition comprises (i) at least a ketone body compound and (ii) a mixture of essential amino acids.

The brain can use ketone body compounds, and its glial cells are also capable of producing ketone. During the treatment, most of the energy needs of the healthy cells are fulfilled by the enteral administration of a nutritional composition comprising at least one ketone body compound, preferably in the form of a physiologically acceptable 3-hydroxybutyrate (BHB), particularly D-3-hydroxybutyrate (or (R)-3-hydoxybutyrate), e.g., a BHB salt such as an alkaline metal, alkaline earth metal or amino acid salt. In certain embodiments, the salt is selected from sodium, potassium, calcium, and magnesium D-3-hydroxybutyrate or a combination thereof. The use of a mixture of those salts may avoid the overloading of sodium while providing the needed amount of butyrate. Administering an amino acid salt, e.g., lysine D-3-hydroxybutyrate, is of special interest since lysine is an antagonist of arginine. In particular embodiments, the composition comprises a mixture of ketone body compounds such as at least 2 or 3 different D-3-hydroxybutyrate salts of sodium, calcium, potassium, magnesium and/or lysine.

In certain embodiments, the ketone body compound may be a BHB ester including but not limited to 3-hydroxybutyl-3-hydroxybutyrate, particularly (R)-3-hydroxybutyl-(R)-3-hydroxybutyrate or ethyl-3-hydroxybutyrate, ethyl-(R)-3-hydroxybutyrate.

In certain embodiments, the content of BHB in the composition is about 0 to about 60 % (w/w) based on the dry weight of the composition.

According to the first and second aspects, the composition comprises a mixture of essential amino acids. In certain embodiments, the composition comprises at least 6, at least 7, at least 8 and particularly 9 amino acids selected from L-valine, L-leucine, L-isoleucine, L-lysine, L-methionine, L-phenylalanine, L-threonine, L-tryptophan, and L-histidine. In certain embodiments, the composition further comprises L-aspartate. Aspartate is a nonessential amino acid but is needed for ASS activity, so it can be admixed to the essential amino acids.

According to the second aspect, the composition may be free from a ketone body compound, e.g., free from a BHB.

The enteral nutritional composition is free from the amino acids that are to be depleted in the subject's body during the treatment. In particular embodiments, the composition is free from L-asparagine, L-arginine, L-citrulline and optionally from L-glutamine.

In certain embodiments, the content of essential amino acids in the composition is about 10 to about 15% (w/w) based on the dry weight of the composition.

The composition may comprise further constituents, e.g. one or more of a carbohydrate source such as glucose or another carbohydrate, e.g., an oligo or polysaccharide which is a source of glucose such as maltose or maltodextrin, a lipid source such as lecithin, a source of fibers, an anti-oxidative agent, a flavoring agent and a salt. Furthermore, the nutritional composition optionally comprises water.

Fiber is routinely added to all compositions for gastric feeding usually as a mixture of water-soluble and water-insoluble types. There are many conventional choices, e.g., pea fibers, acacia gum, fructo-oligosaccharides, inulin, and/or methylcellulose. Examples of anti-oxidative agents are vitamin E compounds, e.g., tocopherol, and/or vitamin C compounds, e.g., E304 (ascorbyl palmitate or ascorbyl stearate). Examples of salts are chloride salts such as sodium, potassium and/or magnesium chloride.

In certain embodiments, the composition is free from a carbohydrate source, particularly free from glucose.

In certain embodiments, the composition comprises a carbohydrate source, particularly glucose or another carbohydrate, e.g., an oligo or polysaccharide which is a source of glucose such as maltose and/or maltodextrin. In particular embodiments, the carbohydrate source comprises or consists of glucose and/or maltodextrin.

During the course of the treatment, a subject may receive at least 2, e.g., 2, 3 or 4 different versions of the nutritional composition, which differ in their contents of ingredients. In certain embodiments, a set of several nutritional compositions is used during the treatment. These compositions may differ with respect to their contents of carbohydrate source, e.g., glucose and/or maltodextrin and/or their weight ratios of ketone body compound, e.g. BHB to carbohydrate source, e.g. glucose and/or maltodextrin. For example, the set may comprise at least 3, e.g. 3 or 4 compositions, e.g., a first composition without a carbohydrate source, which is used during the first phase of the treatment, a second composition having a weight ratio of ketone body compound, e.g. BHB to carbohydrate source between about 1:1 to about 10:1, e.g., about 2:1 to 3:1, which is used during a second phase of the treatment, a third composition having a weight ratio of ketone body compound, e.g. BHB to carbohydrate source between about 1:2 to about 1:20, e.g., about 1:5 to 1:10, which is used during a third phase of the treatment, and optionally a fourth composition without ketone body compound.

The nutritional composition is administered to the subject in need thereof in an amount to maintain metabolic balance throughout the amino acid depletion treatment. An average dog patient may need a total caloric value of about 500 to about 1000 kcal per day, e.g., about 750 kcal per day. An average human patient may need a total caloric value of about 800 to about 2000 kcal per day, e.g., about 1500 kcal per day.

The inventors have found that systemic delivery of an arginine-decomposing enzyme such as an arginase (ARG) and a citrulline-converting enzyme such as argininosuccinate synthase (ASS) in partially purified liver extracts prepared by low-temperature protocols has led to a reduction of free plasma arginine to below detection levels. This was the basis of co-owned application WO 2023/066910, supra, describing a medicament comprising an arginine-decomposing enzyme such as an ARG and a citrulline-converting enzyme such as an ASS for use in a deep depletion of arginine levels in circulating blood.

In certain cases, enzymatic decomposition of arginine by e.g., an arginase (ARG) was found to be necessary but not sufficient for a deep, systemic depletion of arginine. In these cases, concurrent removal of citrulline on its passage from the intestines, the main organ for citrulline synthesis, to kidneys where citrulline is converted to arginine, by a citrulline-converting enzyme, e.g., argininosuccinate synthase (ASS) is required. This enables systemic reduction of free arginine to a concentration below detection by standard amino acids analysis and leads to the rapid killing of cancerous cells of many if not all cancer types. Healthy dividing cells respond by exiting into G₀ phase and very few suffer terminal damage.

In certain embodiments, the arginine-decomposing enzyme is an arginase (ARG), i.e., an enzyme, which catalyzes the hydrolysis of L-arginine to L-ornithine and urea (EC. 3.5.3.1). Typically, the arginase is a mammalian arginase, e.g., a human arginase including any enzymatically active fragment and derivative thereof. In particular embodiments, the arginase is selected from human arginase-1 (liver arginase or ARG1) (UniProt-P05089) or human arginase 2 (kidney arginase or ARG2) (UniProt-P78540) including any enzymatically active fragment and derivative thereof. In even more particular embodiments, the arginase is human ARG1.

Human ARG1 is a 322 amino acid-long polypeptide with a predicted molecular weight of 34,735 Da. It may be present in a monomeric or multimeric, e.g., trimeric form. Preferably, it is present in a monomeric form, which is the native form in the liver (personal communication from Prof. M. Ikemoto, U. of Kyoto, who was the first to clone ARG1). The preparation of recombinant ARG1 in E. *coli* is described by Ikemoto et al. in: Expression of human liver arginase in Escherichia coli. Purification and properties of the product. Biochem J. 1990 Sep 15;270(3):697-703. doi: 10.1042/bj2700697. PMID: 2241902; PMCID: PMC1131788; the content of which is herein incorporated by reference.

In certain embodiments, the arginase is a recombinant human ARG1 including any enzymatically active fragment and derivative thereof. In particular embodiments, the arginase is an unPEGylated polypeptide, i.e., it is not conjugated to a polyethylene glycol moiety.

A modification of human ARG1 to replace manganese with cobalt and to shift the optimum pH to that of plasma is also particularly suitable according to the present invention (Stone EM, Glazer ES, Chantranupong L, et al. Replacing Mn(2+) with Co(2+) in human arginase enhances cytotoxicity toward I-arginine auxotrophic cancer cell lines [published correction appears in ACS Chem Biol. 2010 Aug 20;5(8):797]. ACS Chem Biol. 2010;5(3):333-342. doi:10.1021/cb900267j), the content of which is herein incorporated by reference.

In certain embodiments, arginase, e.g., human ARG1 is administered as an arginase-insulin fusion protein as described in co-owned application WO 2023/041758, the content of which is herein incorporated by reference.

In another particular embodiment, the arginine-decomposing enzyme is an arginine deiminase, e.g., an arginine deiminase of 46 kDa (UniProt-A0A0C6G6L6_MYCAR).

In another particular embodiment, the arginine-decomposing enzyme is an arginine decarboxylase, e.g., a biosynthetic arginine decarboxylase of 74 kDa (UniProt-Q8FE34 SPEA_ECOL6) or a biodegradative arginine decarboxylase of 80 kDa (UniProt- A0A376VTJ3_ECOLX).

Arginase may be supplied externally, but an alternative is its endogenous release from the liver which would, due to low glucose, undergo a degree of necrosis and/or apoptosis. Such methods, e.g. as described in EP 24182531.4 and EP 24186856.1, supra, involve targeted disruption of liver cells by infusing a hypotonic fluid, e.g., water for injection into the hepatic vein of a liver segment, or by inducing liver reperfusion injury by temporary occlusion of a hepatic vein of a liver segment.

In certain embodiments, the citrulline-converting enzyme is an argininosuccinate synthase (ASS), i.e., an enzyme, which catalyzes the conversion of L-aspartate and L-citrulline to L-argininosuccinate (EC 6.3.4.5). Typically, the ASS is a mammalian ASS, e.g., a human ASS including any enzymatically active fragment and derivative thereof. In particular embodiments, the ASS is human argininosuccinate synthase (ASS1) (UniProt-P00966) including any enzymatically active fragment and derivative thereof.

Human ASS1 is a 412 amino acids long polypeptide with a predicted molecular weight of 46,530 Da. It may be present in a monomeric or multimeric, e.g., tetrameric form. Preferably, it is present in a tetrameric form, which is its native active form in the human liver.

In certain embodiments, the ASS is a recombinant human ASS1 including any enzymatically active fragment and derivative thereof. In certain embodiments, the ASS is a PEGylated polypeptide. In other embodiments, the ASS is an unPEGylated polypeptide.

In certain embodiments, ASS is needed to bring arginine to a very low concentration. It also is released by the liver due to the gradual, partial loss of hepatocytes deprived of glucose. ASS may be supplied externally, but an alternative is its endogenous release from the liver as described in EP 24182531.4 and EP 24186856.1, supra.

In certain embodiments, ARG and ASS are present as at least partially purified mammalian liver extract. A preferred method for obtaining a suitable liver extract is described in the Examples of the co-owned application WO 2023/066910, supra. In certain embodiments, ARG and ASS are recombinant polypeptides.

In certain embodiments, both ARG and ASS are supplied by external administration, e.g., infusion.

In certain embodiments, an external administration of ARG and/or ASS is not required.

In a particular embodiment, the release of endogenous amino acid degrading enzymes, particularly ARG and ASS, from disrupted liver cells is effected by liver reperfusion injury caused by temporary occlusion of a hepatic vein of a liver segment. In this embodiment, a nutritional composition comprising a mixture of essential amino acids, particularly 9 essential amino acids optionally together with L-aspartate and without a ketone body compound, e.g., without a BHB, may be used.

Another aspect of research by the inventors was the modification of the delivery of L-asparaginase (ASNase). ASNase can be purified from certain strains of E. *coli* or can alternatively be produced by recombinant technology from genetically modified strains of E. *coli.* ASNase is a commonly used drug to treat blood cancers, e.g., leukemias and lymphomas.

In a particular embodiment, the ASNase is in its tetrameric (active) form of 140 kDa, or in its monomeric form of 35 kDa.

In an even more particular embodiment, the ASNase is in a dissociated form, particularly in the form of a monomer, as described in co-owned application WO 2020/245041. The reversible dissociation of ASNase into its monomers by a high concentration of a chaotropic agent such as urea as a functional excipient facilitates the extravasation of monomers which are then reconstituted in the interstitial fluid in the surrounding of most of the cancer cells of any type of cancer, including blood cancers, as disclosed in the co-owned application WO 2020/245041, supra.

The asparaginase may be administered as an aqueous preparation comprising urea, particularly in a concentration between about 3 mol/l to about 8 mol/l, more particularly in a concentration of about 4 mol/l to about 6 mol/l, e.g., about 5 mol/l.

In certain embodiments, glutaminase (GLS) is administered, particularly in combination with ASNase and optionally ARG and/or ASS. GLS can be added should ASNase fail to sufficiently lower glutamine. In dogs treated with asparaginase, glutamine concentration could be lowered to between the detection limit (<1 micromolar) and 100 micromolar, from the normal level of close to 1 mM. An effective reduction calls for glutamine of < 50 micromolar, preferably of < 10 micromolar.

In certain embodiments, the GLS is a kidney-type GLS, e.g., human kidney (GLSK) with 669 amino acids and a molecular weight of 73,461 Da or any processed form thereof such as Isoform 3 of 68 kDa, or a liver-type GLS, e.g., human liver GLS (LGA)as described by Katt et al (Future Med Chem 2017, 9(2), 223-243, and 9(5), 527), the content of which is herein incorporated by reference In certain embodiments, the GLS is a recombinant polypeptide.

Further, the present invention may comprise the administration of a blood glucose-lowering agent, e.g., an insulin or an insulinotropic peptide, particularly a fast-acting insulin. Insulin is a growth factor that also is a glucose-lowering hormone.

In certain embodiments, the insulin is administered as an arginase-insulin fusion protein as described in co-owned application WO 2023/041758, supra.

The treatment according to the present invention may be carried out under conditions of glucose depletion. Glucose depletion is understood as a blood glucose level of lower than the normal glucose level of the subject to be treated. In certain embodiments, conditions of glucose depletion involve adjusting and maintaining a blood glucose level of about 2 mM or less, preferably as low as about 1 mM. Glucose depletion may be achieved by administration of a blood glucose-lowering agent as described herein.

In certain embodiments, conditions of glucose depletion are maintained throughout a treatment cycle, e.g., during the administration of the amino acid-degrading enzyme. In certain embodiments, the conditions of glucose depletion are maintained for at least 12 h, at least 24, at least 48 h, or at least 72 h.

In further embodiments, conditions of glucose depletion are maintained at the beginning of a treatment cycle. In certain embodiments, the conditions of glucose depletion are maintained for up to 12 h, up to 24 h, up to 48 h, or up to 72 h.

In certain embodiments, the blood glucose-lowering agent is administered without concomitant administration of glucose or a glucose precursor, e.g., a glucose-containing oligo or polysaccharide such as maltose and/or maltodextrin.

A particular aspect of the invention relates to a medicament, which comprises or consists of a combination of the following active agents:
(i) a nutritional composition for enteral administration comprising at least one ketone body compound, e.g., a physiologically acceptable 3-hydroxybutyrate and a mixture of essential amino acids, particularly 9 essential amino acids optionally together with L-aspartate;
(ii) a blood glucose-lowering agent, e.g., an insulin;
(iii) an arginine-decomposing enzyme such as an ARG;
(iv) a citrulline-converting enzyme such as an ASS, and
(v) an asparagine-decomposing enzyme such as an ASNase;
(vi) optionally a glutamine-decomposing enzyme such as a GLS.

A further particular aspect of the invention relates to a medicament which comprises or consists of a combination of the following active agents:
(i) a nutritional composition for enteral administration comprising a mixture of essential amino acids, particularly 9 essential amino acids optionally together with L-aspartate;
(ii) a blood glucose-lowering agent, e.g., an insulin;
(iii) an arginine-decomposing enzyme such as an ARG;
(iv) a citrulline-converting enzyme such as an ASS, and
(v) an asparagine-decomposing enzyme such as an ASNase;
(vi) optionally a glutamine-decomposing enzyme such as a GLS.

In certain embodiments, ARG and/or ASS need not be added externally but are generated endogenously as described above.

The medicament may be comprised of several pharmaceutical preparations, e.g. comprising each active agent separately. Alternatively, the medicament may be a combination of separate pharmaceutical preparations, e.g., one comprising ASNase dissolved in urea, the other comprising ARG and ASS, and the third a nutritional composition as described above.

The medicament of the invention is suitable for use in human medicine and veterinary medicine, e.g., for the treatment of non-human animals, particularly dogs. While the use of the human versions of ARG and ASS is acceptable in treating dogs, canine versions of ARG and ASS are preferred. Both have been produced as recombinant proteins expressly for use in dogs.

Administration of the medicament in a therapeutically active dose will lead to a deep depletion of arginine levels in the blood. In certain embodiments, the arginine levels are about 20 µM or less for a time of at least 12 h or even for at least 72 h, preferably the arginine levels are about 10 µM or less for a time of at least 12 h or even for at least 72 h, more preferably the arginine levels are about 5 µM or less for a time of at least 12 h or even for at least 72 h, and most preferably the arginine levels are about 1 µM or less for a time of at least 12 h or even for at least 72 h as measured in the venous blood plasma. Citrulline concentration in the human plasma of healthy individuals is 30 to 50 µM. Observations from human patients with advanced hepatocellular carcinoma that the inventors participated in treating with arterial occlusion of the liver suggest that lowering citrulline level two to five-fold is sufficient to lower arginine level to the target of less than 1 µM. In dogs normal citrulline concentration is about two times higher than in humans but the same reduction of two to five times was correlated with successful arginine depletion.

In certain embodiments, the medicament of the present invention comprises six active agents. The first is a nutritional composition as described above comprising a mixture of essential amino acids and optionally a ketone body compound, preferably D-3-hydroxybutyrate or a physiologically acceptable salt thereof such as sodium D-3-hydroxybutyrate. The second active agent is a blood glucose-lowering agent such as insulin. Four further agents are amino acid degrading enzymes, one or two of which (ARG and ASS) may be added externally or released endogenously from the liver deprived of the minimum required circulating glucose. L-asparaginase (ASNase) in all cases is supplied externally by infusion. The sixth active agent is a glutamine-decomposing enzyme such as a GLS. The active agents are administered together with pharmaceutically acceptable excipients, e.g., selected from buffers, salts, and/or stabilizers.

The medicament may be a pharmaceutical preparation comprising each active agent separately. Alternatively, the medicament may be a combination of separate pharmaceutical preparations, e.g., one comprising ASNase dissolved in urea, the other comprising ARG and ASS, and the third comprising a nutritional composition as described above comprising a mixture of essential amino acids and optionally a ketone body compound. Chlorine may be added as magnesium chloride. Glucose may also be mixed with the above, as well as a source of lipids, e.g. lecithin.

The medicament is administered in therapeutically effective doses of the active agents.

Asparaginase (ASNase) in conventional clinical use for blood cancers in people and in dogs is typically given at a dose of 400 IU/kg, 3 times a week for about 3 months. Our own research has demonstrated that this is greatly inadequate. To reach the levels of the enzyme in the interstitial fluid (ISF) needed to exert a relevant effect on the cancer cells based on what is known from the dose-response studies *in vitro* we have been using in experimental and clinical studies in animals 3000 IU/kg. To maintain the enzymatic activity in ISF a daily dose of at least about 1000 IU/kg, e.g., about 3000 IU/kg, should be delivered for the duration of the treatment of 2 to 4 days and no longer than 6 days depending on the type of cancer.

In certain embodiments of the invention, the systemic concentration of glucose is lowered by infusion of insulin. The goal is to lower plasma glucose concentration to the minimum level needed to supplement ketone bodies delivered by infusion. In healthy individuals on a regular diet, the glucose level is about 5 mM while the ketone level is about 0.5 to 1 mM. The ratio of glucose to ketone (so-called glucose-ketone index, or GKI) is thus about 5 to 10. Infusion of ketone can safely bring its plasma concentration to 4 or 5 mM. Insulin should be used to lower glucose to below about 2 mM, preferably to as low as about 1 mM, inverting the ratio of the two basic sources of energy. Thus, in certain embodiments, GKI in blood is about 1 or less, e.g., about 0.1 to about 0.5.

The insulin may be administered by infusion at a predetermined dose rate, e.g., at a predetermined constant dose rate. For humans, the insulin may be administered at a dose rate from about 0.2 to about 2 IU/kg body weight/day, preferably from about 0.5 to about 1.5 IU/kg body weight//day, more preferably about 1 IU/kg body weight//day. For dogs, the insulin may be administered at a higher dose rate from about 0.4 to about 4 IU/kg body weight//day, preferably from about 1 to about 3 IU/kg body weight//day, more preferably about 2 IU/kg body weight//day.

The insulin may be any type of natural or recombinant insulin or insulin analog. Preferably, the insulin is a rapid-acting insulin or a short-acting insulin, more preferably a rapid-acting insulin. Examples of rapid-acting insulin are insulin lispro, insulin aspart or insulin glulisine. Examples of short-acting insulins are regular insulin or insulin velosulin. An alternative is s.c. delivery of slow-acting insulins.

In certain embodiments, the therapeutically effective daily dose of an arginase, e.g., ARG1, in a dog is from about 300 to about 6000 IU/kg/day, preferably from about 1000 to about 4500 IU/kg/day, and most preferably 3000 IU/kg/day as determined by experimental work *in vivo.* For humans, a therapeutically effective daily dose is about 150 to about 3000 IU/kg/day, preferably about 500 to about 2000 IU/kg/day, and most preferably about 1500 IU/kg/day. With a specific activity of recombinant ARG1 of about 1000 IU/mg of protein a preferred therapeutically effective daily dose in a dog is about 0.3 to about 6.0 mg/kg/day and in a human about 0.15 to about 3.0 mg/kg/day.

In certain embodiments, the therapeutically effective daily dose of an ASS, e.g., ASS1, in a dog is from about 0.3 to about 6 IU/kg/day, preferably from about 1 to about 4.5 IU/kg/day, and most preferably 3 IU/kg/day. For humans, a therapeutically effective daily dose is about 0.15 to about 3 IU/kg/day, preferably about 0.5 to about 2 IU/kg/day, and most preferably about 1.5 IU/kg/day. With a specific activity of recombinant ASS1 of about 1 IU/mg of protein a preferred therapeutically effective daily dose in a dog is about 0.3 to about 6.0 mg/kg/day and in a human about 0.15 to about 3.0 mg/kg/day.

In certain embodiments, the dose is adapted based on the measurement of arginine and/or citrulline levels in the blood of the subject to be treated. In particular embodiments, the arginine and/or citrulline levels are measured in the plasma of the venous blood.

Amino acid degrading enzymes are typically administered parenterally, e.g., by injection, or preferably by infusion over a suitable period, for example, over a period from several hours, e.g., at least about 2 hours, or about 6 hours, or even continuously for 1 day up to 6 days, depending on the type of cancer being treated.

Administration of the medicament may be accompanied by certain measures to compensate for side-effects of arginine depletion such as infusion of a nitric oxide (NO) donor, e.g., sodium nitroprusside (SNP) or nitroglycerin, and/or a pressor peptide, e.g., a vasopressin, to balance NO-induced vasodilation. Arginine is the only precursor for the synthesis of short-lived NO. All pressor peptides contain arginine and are short-lived. Co-infusion of Iloprost, a prostacyclin analog has also been found useful in the maintenance of thrombocytes. Infusion of glucose is used only in an emergency should any clinical signs of its lack become manifest.

The medicament is useful for the treatment of cancer including blood cancers or solid cancers particularly selected from hepatocellular carcinoma, melanoma, colon carcinoma, leukemia, lymphoma, osteosarcoma, soft tissue sarcoma, mast cell tumor, pancreatic cancer, lung cancer, brain cancer, and breast cancer. The medicament is also useful for the treatment of cancer metastases, which are disseminated within the patient's body.

The inventors have found that the efficacy of the medicament may be increased by assisting the extravasation of arginase, i.e., the transport from the vascular system into the interstitial fluid. Rapid extravasation of ARG1 in its monomeric form, facilitated by insulin, prevents its elimination by glomerular filtration as described in co-owned application WO 2022/034218 the content of which is herein incorporated by reference.

The target compartment for ARG and ASNase is the interstitial volume. This is where most cancer cells reside - only a small fraction of cancer cells are found in the blood at any stage of the disease, even in blood cancers. Enzymatic activity confined to the vascular system cannot efficiently reduce concentrations of amino acids in extravascular volume to very low levels because of the constant influx of amino acids from protein breakdown under homoeostatic mechanisms. The mass transport between intravascular and interstitial fluid (in both directions) is limited by diffusion and modest convection, the rates of which are simply too low when, e.g., arginine is to be systemically reduced into the µM range. By contrast, ASS is preferably retained in the blood to intercept the transport of citrulline from the intestines to the kidneys. Its large molecular weight of 186 kDa helps to keep it in the vascular system. Optionally, it could be PEGylated.

It is worth noting that all current clinical trials with arginine depletion as a modality for cancer treatment are conducted with PEGylated enzymes (arginase or arginine deiminase) with high molecular weights that make extravasation extremely limited. This prevents deep systemic arginine depletion needed to kill disseminated cancer. There is also a rapid transition to PEGylated ASNase in some of the markets. This is a very unfortunate, misguided development that makes the problem of ASNase underdosing even worse.

A standard clinical practice - both human and veterinary - is to use antihistamines to reduce the risk of allergic reactions to large proteins such as amino-degrading enzymes. Unfortunately, antihistamines strongly reduce vascular permeability and thus, extravasation of these enzymes. The inventors have shown in animal trials that antihistamines negate the positive effect of insulin on extravasation but also that allergic reactions are unlikely if the enzymes are delivered i.v. in high doses. This particularly is the case with asparaginase.

Further, the present invention is explained in more detail by the following examples.

### Examples - Detailed compositions for enteral nutrition

### Version A

### Grams per 100 g

| | | |
|---|---|---|
| | 1. D-Sodium 3-hydroxybutyrate (NaC₄H₇O₃) | 3.30 g |
| | 2. D-Calcium 3-hydroxybutyrate (CaC₈H₁₄O₆) | 6.75 g |
| | 3. D-Potassium 3-hydroxybutyrate (KC₄H₇O₃) | 3.70 g |
| *Total of BHBs* | | *13.75 g* |
| | 4. MgCl₂ | 1.20 g |
| | 5. L-Valine | 0.55 g |
| | 6. L-Leucine | 0.91 g |
| | 7. L-Isoleucine | 0.41 g |
| | 8. L-Lysine HCl | 0.59 g |
| | 9. L-Methionine | 0.37 g |
| | 10. L-Phenylalanine | 0.17 g |
| | 11. L-Threonine | 0.50 g |
| | 12. L-Tryptophan | 0.09 g |
| | 13. L-Histidine | 0.17 g |
| | 14. L-Aspartate | 0.24 g |
| *Total of amino acids* | | *4.00 g* |
| | 15. Lecithin | 12.50 g |
| | 16. Fiber | 1.00 g |
| | 17. Antioxidant(s) | |
| | 18. Vitamins | |
| | 19. Water | 67.55 g |

### Version B

### Grams per 100 g

| | | |
|---|---|---|
| | 1. D-Sodium 3-hydroxybutyrate (NaC₄H₇O₃) | 3.30 g |
| | 2. D-Calcium 3-hydroxybutyrate (CaC₈H₁₄O₆) | 6.75 g |
| | 3. D-Potassium 3-hydroxybutyrate (KC₄H₇O₃) | 3.70 g |
| *Total of BHBs* | | *13.75 g* |
| | 4. MgCl₂ | 1.20 g |
| | 5. Glucose | 5.0 g |
| | 6. L-Valine | 0.55 g |
| | 7. L-Leucine | 0.91 g |
| | 8. L-Isoleucine | 0.41 g |
| | 9. L-Lysine HCl | 0.59 g |
| | 10. L-Methionine | 0.37 g |
| | 11. L-Phenylalanine | 0.17 g |
| | 12. L-Threonine | 0.50 g |
| | 13. L-Tryptophan | 0.09 g |
| | 14. L-Histidine | 0.17 g |
| | 15. L-Aspartate | 0.24 g |
| *Total of amino acids* | | *4.00 g* |
| | 16. Lecithin | 10.00 g |
| | 17. Fiber | 1.00 g |
| | 18. Antioxidant(s) | |
| | 19. Water | 65.05 g |

### Version C

### Grams per 100 g

| | | |
|---|---|---|
| | 1. D-Sodium 3-hydroxybutyrate (NaC₄H₇O₃) | 0.33 g |
| | 2. D-Calcium 3-hydroxybutyrate (CaC₈H₁₄O₆) | 0.68 g |
| | 3. D-Potassium 3-hydroxybutyrate (KC₄H₇O₃) | 0.37 g |
| | *Total of BHBs* | *1.38 g* |
| | 4. NaCl, CaCl₂, KCl, MgCl₂ (combined) | 1.20 g |
| | 5. Glucose | 13.0 g |
| | 6. L-Valine | 0.55 g |
| | 7. L-Leucine | 0.91 g |
| | 8. L-Isoleucine | 0.41 g |
| | 9. L-Lysine HCl | 0.59 g |
| | 10. L-Methionine | 0.37 g |
| | 11. L-Phenylalanine | 0.17 g |
| | 12. L-Threonine | 0.50 g |
| | 13. L-Tryptophan | 0.09 g |
| | 14. L-Histidine | 0.17 g |
| | 15. L-Aspartate | 0.24 g |
| *Total of amino acids* | | *4.00 g* |
| | 16. Lecithin | 10.00 g |
| | 17. Fiber | 1.00 g |
| | 18. Antioxidant(s) | |
| | 19. Water | 69.42 g |

### Version D

### Grams per 100 g

| | | |
|---|---|---|
| | 1. Glucose | 15.0 g |
| | 2. L-Valine | 0.55 g |
| | 3. L-Leucine | 0.91 g |
| | 4. L-Isoleucine | 0.41 g |
| | 5. L-Lysine HCl | 0.59 g |
| | 6. L-Methionine | 0.37 g |
| | 7. L-Phenylalanine | 0.17 g |
| | 8. L-Threonine | 0.50 g |
| | 9. L-Tryptophan | 0.09 g |
| | 10. L-Histidine | 0.17 g |
| | 11. L-Aspartate | 0.24 g |
| *Total of amino acids* | | *4.00 g* |
| | 12. Lecithin | 10.00 g |
| | 13. Fiber | 1.00 g |
| | 14. NaCl, CaCl₂, KC, MgCl₂ (combined) | 1.20 g |
| | 15.Antioxidant(s) | |
| | 16. Water | 68.80 g |

All four versions have approximately the same caloric value of 150 kcal/100 g. An average dog may need 500 g (approximately 500 ml) of either of the above three compositions with a total caloric value of 750 kcal per day.

An average human patient may need 1000 ml with approximately 1500 kcal per day.

In the above compositions, BHB salts can be replaced partially or fully by an ester of beta-hydroxybutyrate, e.g., by 3-hydroxybutyl-3-hydroxybutyrate or ethyl-3-hydoxybutyrate, increasing their contents by approximately 40% and decreasing the water content appropriately.

Version A is devoid of any carbohydrates. Insulin further depresses the systemic level of glucose, so metabolic energy for the body is provided almost exclusively by enterally provided D-3-hydroxybutyrates. Cancerous cells cannot substitute hydroxybutyrate for glucose and will be quickly eliminated. However, liver cells also need glucose, and with Version A composition, they will undergo gradual necrosis, releasing all enzymes needed for systemic depletion of arginine as the main, synergistic attack on cancer. Monitoring liver enzymes can be used to switch from Version A to Version B, with a balanced ratio of glucose to hydroxybutyrate. Finally, the treatment can be continued with Version C, with a high ratio of glucose to hydroxybutyrate, and then optionally Version D, without hydroxybutyrate. Each period is approximately one to two days in duration but is to be adjusted to each patient being treated.

## Claims

1. A nutritional composition for enteral administration for use in a method of treating cancer by amino acid depletion, wherein the composition comprises a mixture of essential amino acids.

2. A nutritional composition for enteral administration for use in a method of treating cancer by amino acid depletion, wherein the composition comprises (i) at least one ketone body compound, e.g., at least one beta-hydroxybutyrate (BHB) and (ii) a mixture of essential amino acids.

3. The composition of claim 1 or 2 for the use of claim 1 or 2, wherein the mixture of essential amino acids comprises at least 6, at least 7, at least 8 and particularly 9 amino acids selected from the group consisting of L-valine, L-leucine, L-isoleucine, L-lysine, L-methionine, L-phenylalanine, L-threonine, L-tryptophan and L-histidine and wherein the composition optionally further comprises L-aspartate.

4. The composition of any one of claims 1-3 for the use of claim 1 or 2, wherein the composition is free from L-asparagine, L-arginine, L-citrulline and optionally from L-glutamine.

5. The composition of any one of claims 1-4 for the use of claim 1 or 2, which further comprises a carbohydrate source, particularly glucose and/or maltodextrin.

6. The composition of any one of claims 1-4 for the use of claim 1 or 2, which is free from a carbohydrate source.

7. The composition of any one of claims 1-6 for the use of claim 1 or 2, wherein the composition is co-administered with at least one further agent selected from a blood glucose lowering agent such as insulin and optionally an NO donor and/or a pressor peptide.

8. The composition of any one of claims 1-6 for the use of claim 1, 2 or 7, wherein the amino acid depletion comprises:
∘ depletion of L-arginine,
∘ depletion of L-arginine and L-citrulline,
∘ depletion of L-asparagine,
∘ depletion of L-asparagine and L-glutamine,
∘ depletion of L-arginine, L-citrulline and L-asparagine,
∘ depletion of L-arginine, L-citrulline, L-asparagine and L-glutamine.

9. The composition of any one of claims 1-6 for the use of claim 1, 2, 7 or 8, wherein the amino acid depletion comprises administration of at least one exogenous amino acid degrading enzyme to the subject, wherein the exogenous amino acid-degrading enzyme is particularly selected from:
∘ an asparaginase, particularly in a dissociated form, more particularly in the form of monomers;
∘ an arginase;
∘ an argininosuccinate synthase;
∘ a glutaminase;
∘ or any combination thereof.

10. The composition of any one of claims 1-6 for the use of claim 1, 2 or 7-9, wherein amino acid depletion comprises disruption of cells within the subject's body thereby releasing an endogenous amino acid degrading enzyme, wherein the endogenous amino acid-degrading enzyme is particularly selected from:
∘ an arginase,
∘ an argininosuccinate synthase;
∘ or any combination thereof.

11. The composition of any one of claims 1-6 for the use of claim 10, wherein a release of endogenous amino acid degrading enzymes, particularly ARG and ASS, from disrupted liver cells is effected by liver reperfusion injury caused by temporary occlusion of a hepatic vein of a liver segment, and wherein the nutritional composition particularly comprises a mixture of essential amino acids without a ketone body compound, particularly without a BHB.

12. A medicament, particularly for use in the treatment of cancer, comprising a combination of the following active agents:
(i) a nutritional composition for enteral administration comprising a mixture of essential amino acids, particularly 9 essential amino acids optionally together with L-aspartate;
(ii) a blood glucose-lowering agent, e.g., an insulin;
(iii) an arginine-decomposing enzyme such as an arginase;
(iv) a citrulline-converting enzyme such as an argininosuccinate synthase,
(v) an asparagine-decomposing enzyme such as an asparaginase; and
(vi) optionally a glutamine-decomposing enzyme such as a glutaminase.

13. A medicament, particularly for use in the treatment of cancer, comprising a combination of the following active agents:
(i) a nutritional composition for enteral administration comprising (i) at least one ketone body compound, e.g., a physiologically acceptable 3-hydroxybutyrate and (ii) a mixture of essential amino acids, particularly of 9 essential amino acids optionally together with L-aspartate;
(ii) a blood glucose-lowering agent, e.g., an insulin;
(iii) an arginine-decomposing enzyme such as an arginase;
(iv) a citrulline-converting enzyme such as an argininosuccinate synthase,
(v) an asparagine-decomposing enzyme such as an asparaginase; and
(vi) optionally a glutamine-decomposing enzyme such as a glutaminase.

14. A method for the treatment of cancer by amino acid depletion, wherein a nutritional composition comprising a mixture of essential amino acids is administered enterally to a subject in need thereof.

15. A method for the treatment of cancer by amino acid depletion, wherein a nutritional composition comprising (i) at least one ketone body compound, e.g., at least one beta-hydroxybutyrate and (ii) a mixture of essential amino acids is administered enterally to a subject in need thereof.
